# EUROPEAN PATENT APPLICATION

(11) **EP 2 508 625 A1**
(43) Date of publication of application: **10.10.2012**
(21) Application number: 11305393.8
(22) Date of filing: 05.04.2011
(51) Int. Cl.: C12Q 1/70

(54) **Real-time duplex PCR for HPV16 and HPV18 simultaneous viral load quantification**

(71) Applicant: Université de Franche-Comté, 25000 Besançon (FR)
(72) Inventor: Pretet, Jean-Luc, 25000 Besançon (FR); Saunier, Maëlle, 71000 Macon (FR); Mougin, Christiane, 25000 Besançon (FR)
(74) Representative: Tetaz, Franck Claude Edouard

(57) **Abstract**

Process, primers and probes for real-time duplex PCR for HPV 16 and HPV 18 simultaneous viral load quantification.

SEQUENCE LISTING

<110>  Université de Franche Comté

<120>  Real-time duplex PCR for HPV16 and HPV18 simultaneous load
       quantification

<130>  69246D28905

<160>  6

<170>  PatentIn version 3.3

<210>  1
<211>  23
<212>  DNA
<213>  Papillomavirus sylvilagi

<400>  1
gagaactgca atgtttcagg acc                                              23


<210>  2
<211>  25
<212>  DNA
<213>  Papillomavirus sylvilagi

<400>  2
tgtatagttg tttgcagctc tgtgc                                            25


<210>  3
<211>  29
<212>  DNA
<213>  Papillomavirus sylvilagi

<400>  3
caggagcgac ccagaaagtt accacagtt                                        29


<210>  4
<211>  25
<212>  DNA
<213>  Papillomavirus sylvilagi

<400>  4
tgtctaagtt tttctgctgg attca                                            25


<210>  5
<211>  29
<212>  DNA
<213>  Papillomavirus sylvilagi

<400>  5
tatggagaca cattggaaaa actaactaa                                        29


<210>  6
<211>  14
<212>  DNA
<213>  Papillomavirus sylvilagi

<400>  6
accgcaggca cctt                                                        14

## Description

The present invention is directed to a process for HPV16 (human papillomavirus genotype 16) and HPV18 (human papillomavirus genotype 18) simultaneous load quantification in biological samples.

Persistent infection with a high risk human papillomavirus (HPV) is the major risk factor for the development of cervical cancer (Wallin, 1999). HPV infection is also the most frequent sexually transmitted infection with more than 70% of women that will be infected during their sexual life. However, most infections will be cleared within three years by the host immune response and cervical cancer represents a rare consequence of an HPV infection.

Amongst the 103 HPV types completely sequenced, some have been classified as low risk HPV as they are rarely associated with cervical cancer; while others are classified as high risk HPV due to their high rate of detection in cervical cancer. In particular, HPV16 and HPV18 are the most prevalent papillomaviruses in cervical cancers and they are associated with the highest risks of developing cancer (Odd ratios of 435 and 248 respectively) (Munoz *et al.,* 2003). Moreover, the 10 year cumulative incidence of high grade precancerous lesions or cancers, referred to as "≥CIN3" (Cervical Intraepithelial Neoplasia grade 3), is much higher for women infected with one of these 2 genotypes (17% for HPV 16 and 14% for HPV18) than for women infected by any other high risk genotype (3%) (Khan *et al.,* 2005). Thus, the detection of these two genotypes in cervical samples might be useful to identify women with the highest risk of developing high grade lesions and cervical cancer.

It has been proposed that the HPV-specific DNA load could be useful as a marker of prevalent and incident precancerous and cancerous lesions of the cervix (Gravitt *et al.,* 2007, Hernandez-Hernandez *et al.,* 2003, Ho *et al.,* 1998, Ikenberg *et al.,* 1997, Melbye *et al.,* 1996, Saunier *et al.,* 2008, Sun *et al.,* 2001, Tsai *et al.,* 2005, van Duin et al. 2002, Monnier-Benoit et al. 2006). For instance, HPV16 DNA load is increasing with lesion severity. Moreover, it has been shown that an HPV16 DNA load higher than 22,000 copies/10³ cells allowed the identification of women with prevalent high grade lesions (Saunier *et al.,* 2008). Furthermore, women with an HPV16 DNA load above 200 HPV16 copies/10³ cells had a higher 18 month cumulative incidence of CIN2/3 than women with viral load below 200 HPV16 DNA copies/10³ cells (Monnier*-Benoit et al.,* 2006).

Finally, two prophylactic vaccines targeting HPV 16 and HPV 18 are now available. These vaccines present a very high efficiency to prevent incident and persistent infections as well as development of intraepithelial lesions associated with the targeted HPV (Rambout CMAJ 2007). There is a need for high-performance assays to track vaccine failure and/or assess vaccine efficiency.

As HPV 16 and HPV 18 are responsible for up to 80% of cervical cancer and as viral load would be a potential marker of precancerous and cancerous lesions, there is a need for tools to evaluate viral load of these two genotypes. These observations underscore the need to develop molecular techniques permitting rapid and accurate detection and quantification of HPV16 and HPV18 DNA from exfoliated cervical cells.

Numerous multiplex PCR, classic or real-time, have been described for HPV detection (Brestovac *et al.,* 2005, Clavel *et al.,* 1998, Gheit *et al.,* 2007, Godfroid *et al.,* 1998, Jeney *et al.,* 2007, Jiang *et al.,* 2006, Lazarus & Caruana, 1996, Mitrani-Rosenbaum *et al.,* 1994, *Nazarenko et al.,* 2008, Nishiwaki *et al.,* 2008, Pizzighella *et al.,* 1995, Schmitt *et al.,* 2006, Schmitt *et al.,* 2008, Soler *et al.,* 1991, Zheng *et al.,* 1995). Only a few authors described multiplex PCR allowing the quantification of the different HPV types (Carcopino *et al.,* 2006, Moberg *et al.,* 2003, Tucker *et al.,* 2001). However, these techniques do not allow for simultaneous quantification of the HPV16 and HPV18 genotypes and setting up such a PCR method represents a technical challenge. Indeed, combining two PCR reactions can lead to a decrease of the sensitivity and/or the specificity of one of the PCR reactions and one target can be preferentially amplified (Polz & Cavanaugh, 1998, Walsh *et al.,* 1992). Preferential amplification could be explained by the properties of the target and the adjacent sequences such as: (i) differences in GC% leading to the faster denaturation of one of the target; (ii) secondary structures that prevent the primers to bind to their target and (iii) copy number of the gene in the studied genome (Elnifro *et al.,* 2000).

The present invention relates to a duplex real-time quantitative PCR method providing simultaneous detection and quantification of the HPV16 and HPV18 genotypes. This process was applied on cervical samples harboring HPV 16 only, HPV 18 only or both types. When comparing the values obtained in duplex PCR with values obtained in simplex PCR we observed that the concordance between the 2 techniques was very strong. HPV16/18 duplex PCR allows the simultaneous quantification of HPV16 and 18 on a wide range of concentrations.

### SUMMARY

The present invention is related to a process for quantification of the viral load of papillomavirus genotype 16 (HPV16) and of the viral load of papillomavirus genotype 18 (HPV18) in a biological sample by duplex quantitative polymerase chain reaction (qPCR) wherein a fragment of the E6 gene of HPV16 is amplified and quantified with the primers of SEQ ID NO: 1-2 and the probe of SEQ ID NO: 3, and a fragment of the E6 gene of HPV18 is amplified and quantified with the primers of SEQ ID NO: 4-5 and the probe of SEQ ID NO: 6.

In preferred embodiments, the probe of SEQ ID NO: 3 and the probe of SEQ ID NO: 6 are both labeled with a different fluorophore/quencher pair.

Preferably, the probe of SEQ ID NO: 3 is labeled with a FAM (6-carboxyfluorescein) fluorophore at the 5' end and with a BHQ-1 (Black Hole Quencher 1) quencher at the 3' end.

Preferably, the probe of SEQ ID NO: 6 is labeled with a VIC fluorophore a the 5' end and a MGB (Minor Groove Binding) quencher at the 3' end.

Preferably, the concentration of the primers of SEQ ID NO:1-2 and of SEQ ID NO: 4-5 is comprised between 300 and 600 nM.

Preferably, the concentration of the probe of SEQ ID NO: 3 is comprised between 200 and 300 nM and the concentration of the probe of SEQ ID NO:6 is comprised between 200 and 300 nM.

In preferred embodiments, the duplex quantitative polymerase chain reaction (qPCR) comprises cycles of 10 to 20 seconds at 95°C and 45 to 60 seconds at 60°C.

Preferably, 35-45 cycles are performed.

Preferably, the biological sample is a cervical sample collected at the endo-exocervix junction.

More preferably, the biological sample is a DNA extract from a cervical sample collected at the endo-exocervix junction.

Another object of the present invention is a kit for quantification of the viral load of papillomavirus genotype 18 (HPV18) in a biological sample comprising, in the same or separate containers, the oligonucleotides of SEQ ID NO: 4-6.

Preferably, the kits comprise the oligonucleotides of SEQ ID NO: 1-6.

Another object of the present invention is an oligonucleotide consisting of a sequence selected from SEQ ID NO: 4-6.

### SEQUENCE LISTING

SEQ ID NO.1: E6 HPV 16 Forward primer
SEQ ID NO: 2: E6 HPV 16 Reverse primer
SEQ ID NO: 3: E6 HPV16 Probe
SEQ ID NO: 4: E6 HPV 18 Forward primer
SEQ ID NO: 5: E6 HPV18 Reverse primer
SEQ ID NO: 6: E6 HPV 18 probe

### DETAILED DESCRIPTION OF THE INVENTION

The present invention is directed to a process for the simultaneous quantification of the viral load of HPV16 and HPV18 in a biological sample by real-time quantitative PCR methods.

Specific selection of primers and probes enables duplex qPCR limiting competition effects between the two PCR reactions and providing reliable quantification of HPV16 and 18 on a wide range of concentrations.

A first object of the present invention is a process for quantification of the viral load of papillomavirus genotype 16 (HPV16) and of the viral load of papillomavirus genotype 18 (HPV18) in a biological sample by duplex quantitative polymerase chain reaction (qPCR) wherein a fragment of the E6 gene of HPV16 is amplified and quantified with the primers of SEQ ID NO: 1-2 and the probe of SEQ ID NO: 3, and a fragment of the E6 gene of HPV18 is amplified and quantified with the primers of SEQ ID NO: 4-5 and the probe of SEQ ID NO: 6.

The terms "viral load" refer to the amount of viral DNA in a biological sample.

The term "biological sample" refers to any solid or liquid material containing or suspected of containing the target nucleic acid or of containing human papillomavirus. The sample may be purified nucleic acids, a biological sample such as a tissue sample, a biological fluid sample or a cell sample. The sample may contain solid or liquid material of human origin.

Preferably, the biological sample is a cervical sample collected at the endo-exocervix junction.

More preferably, the biological sample is a DNA extract from a cervical sample collected at the endo-exocervix junction.

The term "duplex qPCR" refers to simultaneous quantification of HPV16 and HPV18 in the same container by quantitative PCR. Duplex qPCR enables simultaneous amplification of a HPV16 specific target DNA and a HPV 18 specific target DNA in one reaction by using two primer pairs. Advantageously, duplex PCR requires fewer reagents as the reaction is carried out in the same container with the same reagents.

The process of the present invention is based on the specific amplification and detection of HPV16 and HPV18 specific target sequences by quantitative real-time PCR. Real-time qPCR is used to amplify and simultaneously quantify HPV16 and HPV18 in a biological sample. It enables both detection and quantification. In real-time quantitative PCR methods the amplified nucleic acids are detected during the amplification reaction which also enables quantification of the target nucleic acid.

The term "primer" refers to an oligonucleotide or to a derivative thereof having or containing a sequence complementary to a target nucleic acid. The primers hybridize to the denatured target nucleic acid through base pairing to initiate the extension reaction catalyzed by the DNA polymerase. The kits, compositions and methods of the present invention preferably use at least two oligonucleotide primers flanking the target nucleic acid and hybridizing to opposite strands of the nucleic acid.

In the methods of the present invention, the primers of the present invention are derived from a polymorphic region of the E6 gene of papillomavirus. Differentiation between the different genotypes of HPV is obtained by amplification of sequences specific of each genotype. The methods of the present invention rely on the selection of specific primers and probes for quantification of HPV16 and HPV18 avoiding any interference between the primers and probes specific for each genotype.

In preferred embodiments, the concentration of the primers of SEQ ID NO:1-2 and of SEQ ID NO: 4-5 is comprised between 300 and 600 nM in the final reaction mixture, more preferably between 450nm and 550nM and most preferably the concentration in the final reaction mixture is 500nM.

The terms "final reaction mixture" refer to a solution comprising all the required components to carry out the reaction including the sample. The reaction mixture is usually prepared by mixing a determined volume of the amplification reaction mixture with a determined volume of sample. The terms "amplification reaction mixture" refer to a solution comprising some or all the required components to carry out the reaction except for the sample. This amplification reaction mixture is usually termed the "MasterMix". The Mastermix consists of some or all the required components to carry out the reaction except for the sample. The Mastermix typically comprises the reaction buffer, a balanced mix of dATP, dCTP, dTTP and dGTP, a thermostable DNA polymerase, probes and primers. The final reaction mixture comprises both the sample containing or suspected of containing the target nucleic acid and the amplification reaction mixture. The final reaction mixture typically has a volume comprised between 5 and 100 µl.

The term "probe" refers to an oligonucleotide which is used to detect the presence of target nucleic acids that are complementary to the sequence of the probe.

The methods of the present invention typically use a labeled probe and more preferably a dual-labeled probe also called TaqMan® probes as described in EP 0 543 942. A fluorophore is attached to the 5'end of the probe and a quencher to the 3'end. During amplification, the probe is hydrolyzed by the 5' nuclease activity of the thermostable DNA polymerase. This type of assay is also called 5'nuclease real-time quantitative PCR. the probe of SEQ ID NO: 3 and the probe of SEQ ID NO: 6 are both labeled with a different fluorophore/quencher pair.

Preferably, the probe of SEQ ID NO: 3 is labeled with a FAM (6-carboxyfluorescein) fluorophore at the 5' end and with a BHQ-1 (Black Hole Quencher 1) quencher at the 3' end.

Preferably, the probe of SEQ ID NO: 6 is labeled with a VIC fluorophore a the 5' end and a MGB (Minor Groove Binding) quencher at the 3' end.

Preferably, the concentration of the probe of SEQ ID NO: 3 in the final reaction mixture is comprised between 200 and 300 nM, more preferably the concentration of the probe of SEQ ID NO: 3 in the final reaction mixture is 250nM.

Preferably, the concentration of the probe of SEQ ID NO: 6 in the final reaction mixture is comprised between 200 and 300 nM, more preferably the concentration of the probe of SEQ ID NO: 6 in the final reaction mixture is 250nM.

The real-time qPCR reaction itself is performed under standard conditions well known to the skilled person.

In preferred embodiments, the duplex quantitative polymerase chain reaction (qPCR) comprises cycles of 10 to 20 seconds at 95°C and 45 to 60 seconds at 60°C.

The methods of the present invention may further comprise an initial inactivation step (1 - 3 minutes at 50°C) to allow the uracyl DNA glycosylase action followed by a denaturation step which is carried out at 94-95°C (5-10 minutes) for complete denaturation of the nucleic acid. This initial step may also serve as a heat activation step for a HotStart DNA polymerase.

Preferably, between 35 and 45 PCR cycles are performed.

Reaction components for nucleic acid amplification are commonly commercialised as kits comprising at least one or more of the reagents/components necessary to carry out the amplification of a target nucleic acid.

Another object of the present invention is a kit for quantification of the viral load of papillomavirus genotype 18 (HPV18) in a biological sample comprising, in the same or separate containers, the oligonucleotides of SEQ ID NO: 4-6.

Another object of the present invention is a kit for quantification of the viral load of papillomavirus genotype 16 (HPV16) and of the viral load of papillomavirus genotype 18 (HPV18) in a biological sample comprising, in the same or separate containers, the oligonucleotides of SEQ ID NO: 1-6.

In another embodiment, the invention is related to an oligonucleotide consisting of a sequence selected from SEQ ID NO: 4-6.

After optimization of primers and probes concentrations, we validated the method on HPV16 and HPV18 plasmid mixes. When comparing values obtained with the theoretical values we observed that when one genotype is in excess of more than 1000 folds, the experimental concentration of the genotype which is in lower concentration can be under-estimated or over-estimated. Similar results were obtained with the multiplex technique developed by Moberg *et al.* which was not quantitative any more when the HPV16/18 ratio is higher than 1:100. These observations can be explained by competition effects between the two PCR. However, when the method is applied on DNA from cervical samples, the variability between results obtained in simplex PCRs and results obtained in duplex PCR is very low in both mono-infected samples (HPV16 or HPV18) and in co-infected samples. Slight limitations have been observed: the duplex PCR is less sensitive than simplex PCR as it does not permits the detection of viral loads smaller than 2 genome equivalents per µL in mono-infected samples and viral loads smaller than 10 ge/µL in co-infected samples. Nevertheless, one can wonder the utility of being able to detect such low viral loads. Furthermore, these values are far below the threshold values we set up for incident (200 HPV16 copies/1000 cells) and prevalent (20,000 HPV16 copies/1000 cells) high grade lesions (Monnier*-*Benoit *et al.,* 2006, Saunier *et al.,* 2008). During validation on plasmid mixes, we showed that when one genotype was in large excess, the viral load of the less represented genotype could be over or under estimated. This observation was not made on cervical samples but only 2 samples presented concentration ratios higher than 1000. In such cases, one can wonder what the clinical signification of the less represented genotype is, and only prospective studies can answer this problematic.

### FIGURES

Figure 1: HPV16 load (ge/µL) (A, C) and HPV18 load (ge/µL) (B, D) determined by simplex (A, B) and duplex (C, D) real-time PCR results from mixes of HPV16 and HPV 18 genome containing plasmids.
Figure 2: Comparison of HPV16ge number (A) and HPV18ge number (B) determined by simplex and duplex real-time PCR on plasmid mixes.
Figure 3: Comparison of HPV16ge number (A) and HPV18ge number (B) determined by simplex and duplex real-time PCR on clinical samples.

### EXAMPLES

### Materials and methods

Quantification of HPV 16 and HPV 18 DNA copy number was performed by real-time PCR with an AB7500 thermocycler (Applied Biosystems). Primers and hydrolysis probes were designed to target a polymorphic region of the E6 gene. Primer and probe sequences are described in table 1. The HPV16-specific hydrolysis probe was labeled with the FAM fluorophore at the 5' end (maximum emission wavelength of 518 nm) combined with the Black Hole quencher 1 at the 3' end. The E6 HPV 18 hydrolysis probe was labeled with the VIC fluorophore on the 5' end (maximum emission wavelength of 555 nm) combined with the Minor Groove Binder (MGB) quencher on the 3' end.

**Table 1: Sequences of primers and probes used for simplex and duplex real-time PCR:**

| **target** | | **Sequence** |
|---|---|---|
| E6 HPV16 | Forward primer | 5'-GAGAACTGCAATGTTTCAGGACC-3' |
| | Reverse primer | 5'-TGTATAGTTGTTTGCAGCTCTGTGC-3' |
| | Probe | 5'-FAM-CAGGAGCGACCCAGAAAGTTACCACAGTT- BHQ1-3' |
| E6 HPV18 | Forward primer | 5'-TGTCTAAGTTTTTCTGCTGGATTCA -3' |
| | Reverse primer | 5'-TATGGAGACACATTGGAAAAACTAACTAA -3' |
| | Probe | 5'-VIC-ACCGCAGGCACCTT-MGB-3' |

### Simplex qPCR targeting E6 HPV16 and E6 HPV18:

Standard curves used to quantify E6 copy number (HPV 16 or HPV18) were made with 6 serial dilutions (1:10) of plasmids containing the whole genome of either HPV16 or HPV18 (pBR322 HPV16 or pBR322 HPV18) diluted in 50 ng/µL salmon sperm DNA. Each simplex reactions was performed in a final volume of 25µL containing 1X Taqman Gene Expression Mastermix (Applied Biosystems), 500nM of each primers (Eurogentec, Seraing, Belgium), 250 nM of probe (Eurogentec, Seraing, Belgium) and 2µL of DNA sample.

These 2 qPCR showed a detection limit of 10 copies/µL.

### Duplex qPCR

Standard curves used to quantify E6 copy number from HPV 16 and HPV 18 were made by mixing serial dilutions (1:10) of HPV16 containing plasmids and HPV18 containing plasmids (equimolar mix). Plasmids were diluted in 50 ng/µL salmon sperm DNA. Duplex reactions were performed in a final volume of 25µL containing 1X Taqman Gene Expression Mastermix (Applied Biosystems), 500nM of each E6 HPV16 primers, 250 nM of E6 HPV16 probe, 500nM of each E6 HPV18 primers, 250 nM of E6 HPV18 probe and 2µL of DNA sample.

### Plasmid mixes

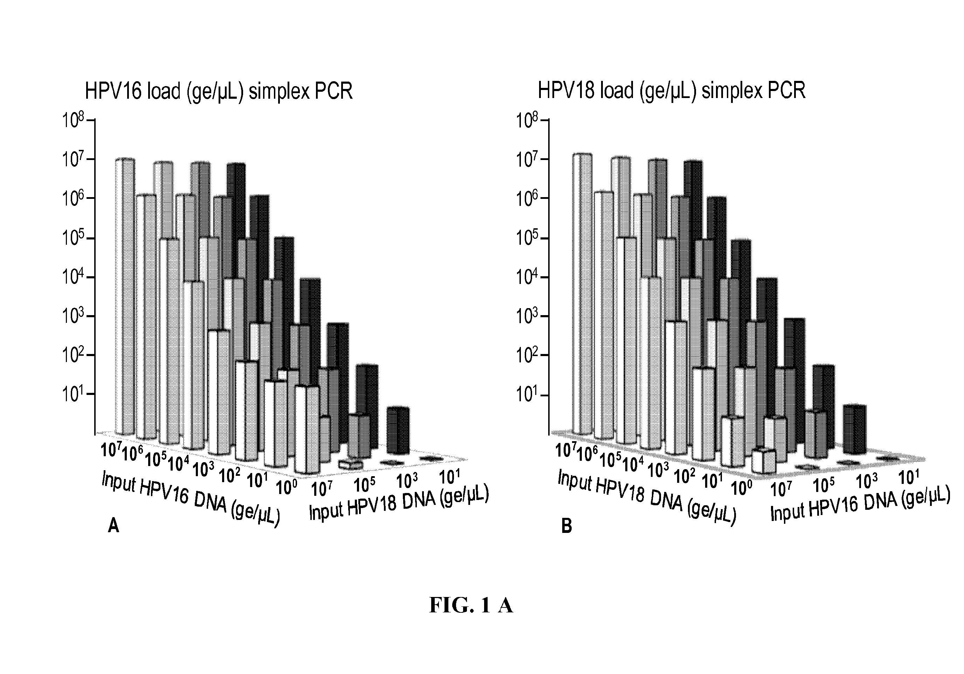

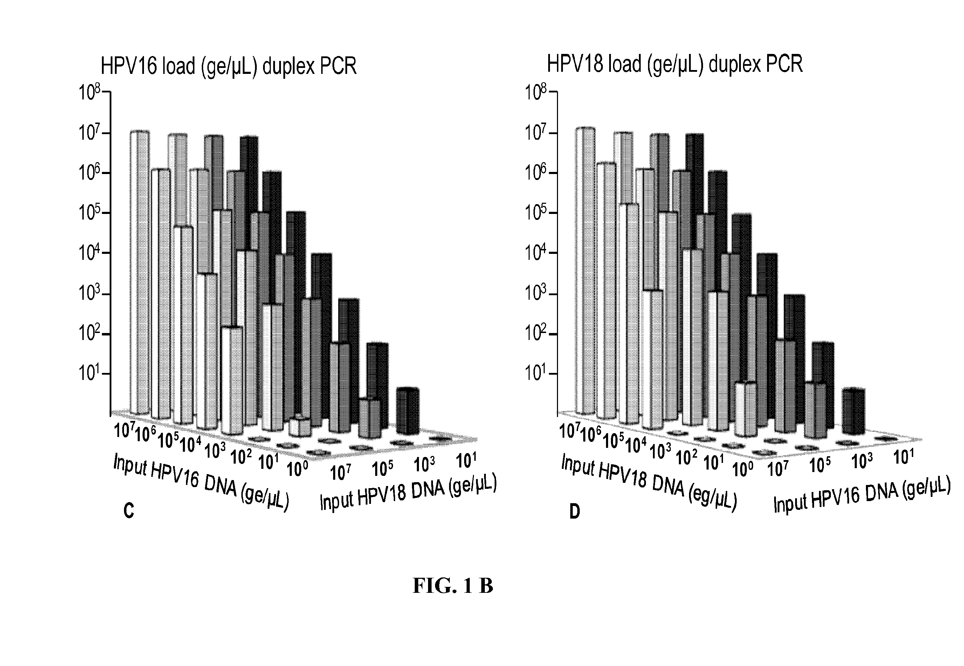

To validate the duplex qPCR, we realized mixes of HPV16 and HPV18 plasmids in various concentrations as described in Figure 1. Mixes were made in a solution containing genomic DNA (C33A cells) corresponding to 20,000 cells/µL, in order to mimic steric hindrance occurring in DNA extracted from clinical samples. Copy number of HPV16 and 18 were expressed as HPV16 or HPV18 genome equivalent (ge).

### Clinical samples

To further validate the duplex PCR technique we used cervical samples collected at the endo-exocervix junction with a cytobrush and stored in PreservCyt (Cytyc, Malborough, USA) or Cytoscreen (Seroa, Monaco, France) (liquid-based cytology transport medium) at 4°C until further processing for routine HR-HPV screening with HC2®. Four mL of samples were centrifuged for 10 min. at 2,500g. After supernatant removal, the cell pellet was suspended in 400 µL Tris-EDTA buffer (pH 8). Then, 400 µL of AL buffer (Qiagen) and 40 µL proteinase K (Qiagen) were added and the cells were digested overnight at 56°C. All lysates were filtered through the column provided in the kit. After washes, DNA was eluted with 80 µL of molecular biology grade water.

After HPV genotyping we selected 37 cervical samples harboring HPV16 and/or HPV18. HPV16 and HPV18 simplex PCR and the HPV16/18 duplex qPCR were applied to these samples and the results were compared. Every PCR was performed twice, in duplicate.

### Results

### Validation on plasmid mixes

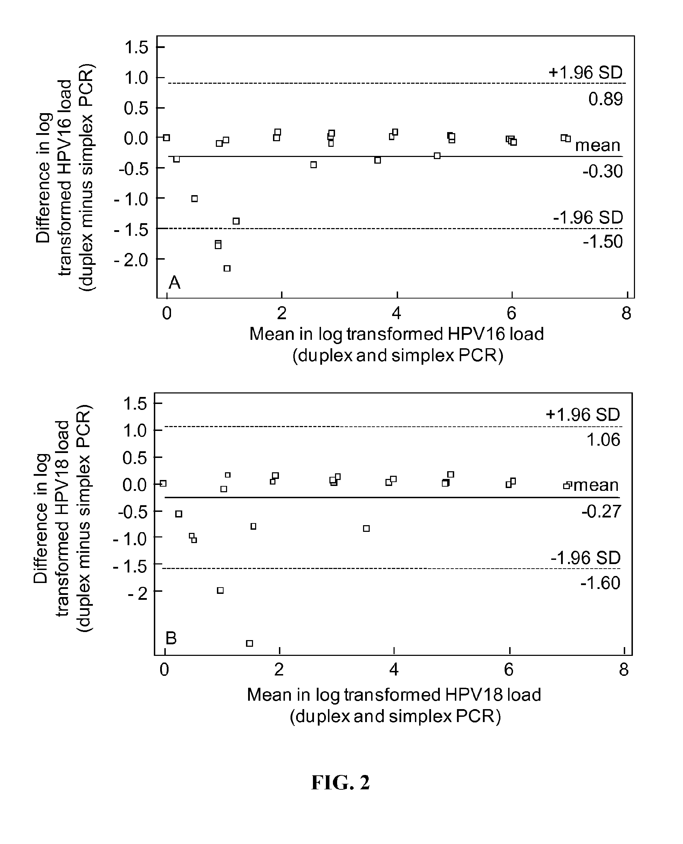

Our first objective was to determine whether simplex real-time PCR permitted to retrieve the correct number of HPV16ge or HPV18ge from mixes with defined HPV DNA titers. As shown in figure 2A, for HPV16, and in Figure 2C for HPV18, the experimental values and the theoretical values were comparable whatever the second HPV genotype concentration was. In particular we did observe a decrease of 1 log in experimental values which is consistent with the decreasing concentration of plasmids obtained after 1/10 serial dilutions. We noted a slight deviation between experimental results and theoretical values for mixes with ≤10 HPV16ge/µL and 10⁷ HPV18ge/µL. In these cases the experimental HPV 16ge reach X and Y HPV 16eg/mL.

Then we plotted the experimental values obtained with the duplex real-time PCR for the quantification of HPV16 DNA (Figure 2B) or HPV18 DNA (Figure 2D) against the theoretical values. For most mixes, experimental results were very close to the expected values. The number of HPV16ge was underestimated by the duplex PCR for mixes with ≤10³ HPV16ge/µL and ≥10⁵ HPV18ge/µL. For HPV18 quantification by duplex PCR, underestimated values were noted for mixes with 10⁷ HPV16ge/µL and ≤10⁴ HPV18ge/µL as well as in samples with 10⁵ HPV16ge/µL and ≤10² HPV18ge/µL.

### Comparison of simplex and duplex real-time PCR for the determination of viral titers from plasmid mixes

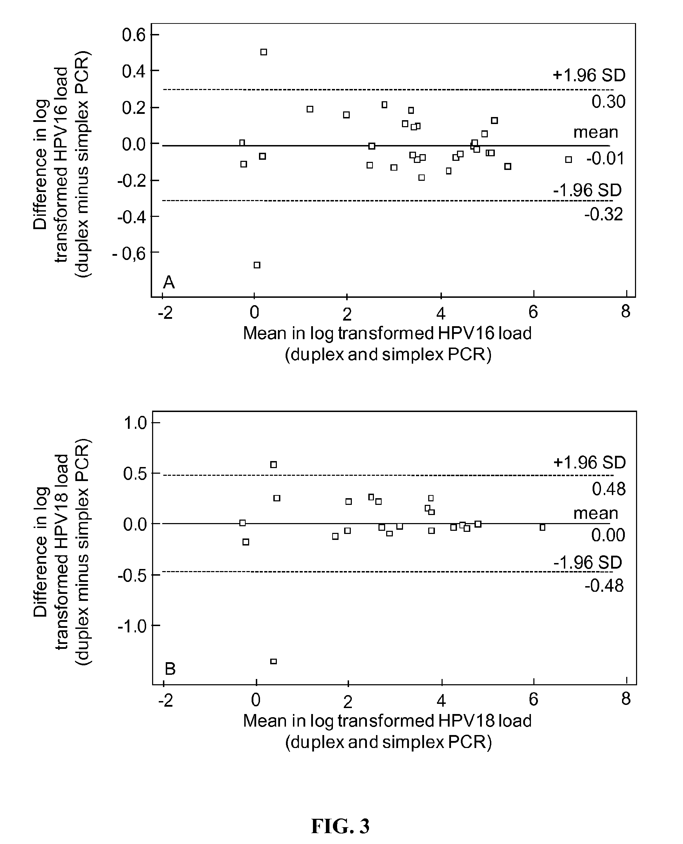

Agreement between experimental results obtained by simplex and duplex real-time was evaluated using Bland-Altman plots (Figure 3). These plots show that 91 % (for HPV16, Figure 3A) and 94% (for HPV18) of the difference scores were between the limit of agreement defined by 1.96 times the standard deviation. The mean difference between logHPV16ge number determined by duplex and simplex PCR was equal to -0.30 and statistically different from zero (95% CI = -0.52 to -0.08). For HPV18, the mean difference was equal to -0.27 and was also different from zero (95% CI = -0.52 to -0.03).

### Comparison of simplex and duplex PCR on clinical samples

In addition, 37 cervical samples positive for HPV16 and/or HPV18 were analyzed by simplex and duplex PCR. Among them 30 were infected by HPV 16, 22 by HPV 18 and 15 were by HPV16 and HPV18. The agreement between the simplex and the duplex PCR was assessed using Bland-Altman plots (Figure 4). First, we noted that 94% (for HPV16) and 95% (for HPV18) of the difference scores were between the limit of agreement corresponding to 1.96 times the standard deviation. Moreover, the means of the differences were not statistically different to zero for both HPV16 (mean = -0.008, 95%CI= -0.056 to 0.039) and HPV18 (mean = -0.001, 95%CI= -0.076 to 0.073).

### REFERENCES

Brestovac, B., Harnett, G. B., Smith, D. W., Frost, F. & Shellam, G. R. (2005). Multiplex nested PCR (MNP) assay for the detection of 15 high risk genotypes of human papillomavirus. J Clin Virol 33, 116-22.
Carcopino, X., Henry, M., Benmoura, D., Fallabregues, A. S., Richet, H., Boubli, L. & Tamalet, C. (2006). Determination of HPV type 16 and 18 viral load in cervical smears of women referred to colposcopy. J Med Virol 78, 1131-40.
Castle PE, Sadorra M, Lau T, Aldrich C, Garcia FA, Kornegay J. Evaluation of a prototype real-time PCR assay for carcinogenic human papillomavirus (HPV) detection and simultaneous HPV genotype 16 (HPV16) and HPV18 genotyping.J Clin Microbiol. 2009 Oct;47(10):3344-7
Gravitt, P. E., Kovacic, M. B., Herrero, R., Schiffman, M., Bratti, C., Hildesheim, A., Morales, J., Alfaro, M., Sherman, M. E., Wacholder, S., Rodriguez, A. C. & Burk, R. D. (2007). High load for most high risk human papillomavirus genotypes is associated with prevalent cervical cancer precursors but only HPV 16 load predicts the development of incident disease. Int J Cancer 121, 2787-93.
Hernandez-Hernandez, D. M., Ornelas-Bernal, L., Guido-Jimenez, M., Apresa-Garcia, T., Alvarado-Cabrero, I., Salcedo-Vargas, M., Mohar-Betancourt, A. & Garcia-Carranca, A. (2003). Association between high-risk human papillomavirus DNA load and precursor lesions of cervical cancer in Mexican women. Gynecol Oncol 90, 310-7.
Ikenberg, H., Goppinger, A., Bauer, H. & Schmitt, B. (1997). Semiquantitative analysis of human papillomavirus DNA in cervical intraepithelial neoplasia by a differential polymerase chain reaction. J Obstet Gynaecol 17, 176-9.
Khan, M. J., Castle, P. E., Lorincz, A. T., Wacholder, S., Sherman, M., Scott, D. R., Rush, B. B., Glass, A. G. & Schiffman, M. (2005). The elevated 10-year risk of cervical precancer and cancer in women with human papillomavirus (HPV) type 16 or 18 and the possible utility of type-specific HPV testing in clinical practice. J Natl Cancer Inst 97, 1072-9.
Moberg, M., Gustavsson, I. & Gyllensten, U. (2003). Real-time PCR-based system for simultaneous quantification of human papillomavirus types associated with high risk of cervical cancer. J Clin Microbiol 41, 3221-8.
Monnier-Benoit, S., Dalstein, V., Riethmuller, D., Lalaoui, N., Mougin, C. & Pretet, J. L. (2006). Dynamics of HPV16 DNA load reflect the natural history of cervical HPV-associated lesions. J Clin Virol 35, 270-7.
Munoz, N., Bosch, F. X., de Sanjose, S., Herrero, R., Castellsague, X., Shah, K. V., Snijders, P. J. & Meijer, C. J. (2003). Epidemiologic classification of human papillomavirus types associated with cervical cancer. N Engl J Med 348, 518-27.
Polz, M. F. & Cavanaugh, C. M. (1998). Bias in template-to-product ratios in multitemplate PCR. Appl Environ Microbiol 64, 3724-30.
Saunier, M., Monnier-Benoit, S., Mauny, F., Dalstein, V., Briolat, J., Riethmuller, D., Kantelip, B., Schwarz, E., Mougin, C. & Pretet, J. L. (2008). HPV16 DNA load and physical state allow the identification of HPV16 infected women with high-grade lesions or cervical carcinoma. J Clin Microbiol. 46(11):3678-85
Schmitz M, Scheungraber C, Herrmann J, Teller K, Gajda M, Runnebaum IB, Dürst M. (2009) Quantitative multiplex PCR assay for the detection of the seven clinically most relevant high-risk HPV types. J Clin Virol. 44:302-7
Sun, C. A., Lai, H. C., Chang, C. C., Neih, S., Yu, C. P. & Chu, T. Y. (2001). The significance of human papillomavirus viral load in prediction of histologic severity and size of squamous intraepithelial lesions of uterine cervix. Gynecol Oncol 83, 95-9.
Tsai, H. T., Wu, C. H., Lai, H. L., Li, R. N., Tung, Y. C., Chuang, H. Y., Wu, T. N., Lin, L. J., Ho, C. K., Liu, H. W. & Wu, M. T. (2005). Association between quantitative high-risk human papillomavirus DNA load and cervical intraepithelial neoplasm risk. Cancer Epidemiol Biomarkers Prev 14, 2544-9.
Tucker, R. A., Unger, E. R., Holloway, B. P. & Swan, D. C. (2001). Real-time PCR-based fluorescent assay for quantitation of human papillomavirus types 6, 11, 16, and 18. Mol Diagn 6, 39-47.

## Claims

1. Process for quantification of the viral load of papillomavirus genotype 16 (HPV16) and of the viral load of papillomavirus genotype 18 (HPV18) in a biological sample by duplex quantitative polymerase chain reaction (qPCR) wherein a fragment of the E6 gene of HPV16 is amplified and quantified with the primers of SEQ ID NO: 1-2 and the probe of SEQ ID NO: 3, and a fragment of the E6 gene of HPV18 is amplified and quantified with the primers of SEQ ID NO: 4-5 and the probe of SEQ ID NO: 6.

2. Process for quantification of the viral load of papillomavirus genotype 16 (HPV 16) and of the viral load of papillomavirus genotype 18 (HPV18) according to claim 1 wherein the probe of SEQ ID NO: 3 and the probe of SEQ ID NO: 6 are both labeled with a different fluorophore/quencher pair.

3. Process for quantification of the viral load of papillomavirus genotype 16 (HPV 16) and of the viral load of papillomavirus genotype 18 (HPV18) according to anyone of claims 1-2 wherein the probe of SEQ ID NO: 3 is labeled with a FAM (6-carboxyfluorescein) fluorophore at the 5' end and with a BHQ-1 (Black Hole Quencher 1) quencher at the 3' end.

4. Process for quantification of the viral load of papillomavirus genotype 16 (HPV 16) and of the viral load of papillomavirus genotype 18 (HPV18) according to anyone of claims 1-3 wherein the probe of SEQ ID NO: 6 is labeled with a VIC fluorophore a the 5' end and a MGB (Minor Groove Binding) quencher at the 3' end.

5. Process for quantification of the viral load of papillomavirus genotype 16 (HPV 16) and of the viral load of papillomavirus genotype 18 (HPV18) according to anyone of claims 1-4 wherein the concentration of the primers of SEQ ID NO:1-2 and of SEQ ID NO: 4-5 is comprised between 300 and 600 nM.

6. Process for quantification of the viral load of papillomavirus genotype 16 (HPV 16) and of the viral load of papillomavirus genotype 18 (HPV18) according to anyone of claims 1-5 wherein the concentration of the probe of SEQ ID NO: 3 is comprised between 200 and 300 nM and the concentration of the probe of SEQ ID NO:6 is comprised between 200 and 300 nM.

7. Process for quantification of the viral load of papillomavirus genotype 16 (HPV 16) and of the viral load of papillomavirus genotype 18 (HPV18) according to anyone of claims 1-6 wherein duplex quantitative polymerase chain reaction (qPCR) comprises cycles of 10 to 20 seconds at 95°C and 45 to 60 seconds at 60°C.

8. Process for quantification of the viral load of papillomavirus genotype 16 (HPV 16) and of the viral load of papillomavirus genotype 18 (HPV18) according to claim 7 wherein 35-45 cycles are performed.

9. Process for quantification of the viral load of papillomavirus genotype 16 (HPV16) and of the viral load of papillomavirus genotype 18 (HPV18) according to anyone of claims 1-8 wherein the biological sample is a cervical sample collected at the endo-exocervix junction.

10. Process for quantification of the viral load of papillomavirus genotype 16 (HPV 16) and of the viral load of papillomavirus genotype 18 (HPV18) according to anyone of claims 1-8 wherein the biological sample is a DNA extract from a cervical sample collected at the endo-exocervix junction.

11. Kit for quantification of the viral load of papillomavirus genotype 18 (HPV18) in a biological sample comprising, in the same or separate containers, the oligonucleotides of SEQ ID NO: 4-6.

12. Kit for quantification of the viral load of papillomavirus genotype 16 (HPV16) and of the viral load of papillomavirus genotype 18 (HPV18) in a biological sample comprising, in the same or separate containers, the oligonucleotides of SEQ ID NO: 1-6.

13. Oligonucleotide consisting of a sequence selected from SEQ ID NO: 4-6.
